Europäisches Patentamt

**European Patent Office**

**Office européen des brevets**

(19)

(11) Numéro de publication: **0 204 638**
**A1**

(12) # DEMANDE DE BREVET EUROPEEN

(21) Numéro de dépôt: **86401204.2**

(22) Date de dépôt: **04.06.86**

(51) Int. Cl.⁴: **A 61 K 7/15**

(30) Priorité: **05.06.85 FR 8508488**

(43) Date de publication de la demande:
**10.12.86 Bulletin 86/50**

(84) Etats contractants désignés:
**AT BE CH DE GB IT LI LU NL SE**

(71) Demandeur: **Valtulina, Arthur**
**Benedikt - Hugiweg 6**
**CH-4143 Dornach(CH)**

(71) Demandeur: **Valtulina, Mario**
**4 Chemin de la Peyriére**
**F-31240 Saint Jean de l'Union(FR)**

(72) Inventeur: **Valtulina, Arthur**
**Benedikt, Hugiweg 6**
**CH-4143 Dornach(CH)**

(72) Inventeur: **Valtulina, Mario**
**4 Chemin de la Peyrière**
**F-31240 Saint Jean de l'Union(FR)**

(72) Inventeur: **Froger, Hubert**
**109 rue du Petit Château**
**F-94220 Charenton(FR)**

(74) Mandataire: **Phélip, Bruno et al,**
**c/o Cabinet Harlé & Phélip 21, rue de la Rochefoucauld**
**F-75009 Paris(FR)**

(54) **Produit de rasage.**

(57) L'invention a pour objet un produit de rasage destiné à être appliqué sur la peau du visage pour faciliter le passage d'un rasoir à lame.

Selon l'invention, le produit est constitué par un elotion liquide contenant un produit capillaire adoucissant du cheveu et ayant de l'affinité pour la kératine, un produit adoucissant de la peau et un produit mouillant.

EP 0 204 638 A1

Croydon Printing Company Ltd

1

L'invention a pour objet un produit de rasage destiné à être appliqué sur la peau du visage pour faciliter le passage d'un rasoir à lame.

Généralement on utilise des crèmes moussantes pour se raser avec un rasoir mécanique à lame. Cet usage est très ancien car on avait remarqué depuis longtemps que la présentation sous forme de mousse permettait de bien mouiller le poil, de faciliter la coupe et d'adoucir la peau. A l'origine, la mousse était réalisée et appliquée avec un blaireau à partir d'un savon doux. Ce mode de préparation, qui est d'ailleurs toujours utilisé, est assez long et demande un matériel relativement encombrant. C'est pourquoi on a mis dans le commerce depuis longtemps des bombes pressurisées permettant d'obtenir directement la mousse. Cependant ces bombes sont toujours assez encombrantes du fait qu'elles doivent contenir une quantité de produit suffisante pour un certain nombre d'utilisations et un gaz propulseur. Cet encombrement peut présenter un inconvénient, par exemple pour le voyage.

On utilise également des crèmes non moussantes fournies par exemple en tubes et qui sont généralement des savons ou émulsions du type huile dans eau. Elles sont assez épaisses et le tube, pour convenir à un nombre raisonnable d'utilisations, reste relativement encombrant.

Dans tous les cas, le produit restant sur le visage après le rasage doit être rincé à l'eau. Le rasage au moyen d'un rasoir à lame est donc toujours une opération relativement longue et qui nécessite l'usage d'un cabinet de toilette. Pour toutes ces raisons, de nombreuses personnes préfèrent utiliser un rasoir électrique, jugé plus pratique, en particulier pour le voyage.

9017 EU

2

L'invention a pour objet un nouveau produit de rasage permettant l'utilisation d'un rasoir à lame mais d'emploi plus simple que les mousses ou crèmes utilisées jusqu'à présent.

Conformément à l'invention, ce produit de rasage est constitué par une lotion liquide contenant un produit capillaire adoucissant du cheveu et ayant de l'affinité pour la kératine, un produit adoucissant de la peau et un produit mouillant.

De façon particulièrement avantageuse, le produit de rasage selon l'invention est constituée par une lotion de traitement des cheveux contenant un produit démêlant.

Dans un mode de réalisation préférentiel, le produit de rasage contient :

- de 4,5 à 5 % d'une cire auto-émulsionnable,
- de 0,2 à 0,5 % d'un polymère quaternaire,
- de 0,5 à 1 % d'un produit tensio-actif,
- au moins 90 % d'eau,
- et un produit épaisssissant en quantité réglée en fonction des proportions des autres constituants de telle sorte que la viscosité du produit de rasage soit comprise entre 50 et 500 centipoises.

L'invention repose essentiellement sur la constatation très surprenante que l'on pouvait utiliser pour se raser avec un rasoir à lame, et même avec un grand confort, une lotion liquide qui, jusqu'à présent, était utilisée pour le traitement des cheveux et notamment pour faciliter le démêlage.

On a observé en effet que, si l'on avait conservé l'habitude d'utiliser des produits à base de savon pour réaliser les mousses et crèmes à raser, ce n'était pas pour nettoyerla peau mais, essentiellement, pour mouiller le poil de façon à le ramollir la mousse ayant, pour une quantité très faible de produit, un pouvoir mouillant important.

Or, les lotions capillaires contiennent toujours un produit mouillant permettant de bien imprégner les cheveux. Par ailleurs, certaines lotions utilisées comme produit de conditionnement des cheveux, par exemple les lotions de protection avant permanente, contiennent des produits substantifs ayant une affinité pour la kératine qui permettent aux produits traitants d'adhérer aux cheveux. Ces produits se fixent sur les cheveux formant une sorte de gaine et ont un effet adoucissant qui facilite le démêlage. On a observé que le poil est de la même nature que le cheveu et que de tels produits mouillants et adoucissants pouvaient, par un effet analogue sur le poil, faciliter le passage du rasoir.

D'autre part, la composition des lotions capillaires est prévue pour que celles-ci n'irritent pas le cuir chevelu et aient un effet adoucissant. Or , surtout lorsque l'on est habitué à se raser avec un rasoir à lame, la peau du visage n'est pas plus fragile que le cuir chevelu et on a observé que, grâce à son effet adoucissant et lubrifiant obtenu par exemple par une cire auto-émulsionnable, une lotion capillaire pouvait avoir un effet bénéfique sur la peau pour empêcher le feu du rasoir.

Bien entendu, on ne peut pas utiliser n'importe quelle lotion capillaire mais l'expérience a montré qu'on obtenait un excellent résultat avec une lotion de traitement ou de conditionnement contenant un produit adoucissant ayant de l'affinité pour la kératine et en particulier un produit démêlant comme un polymère quaternaire, par exemple une protéine quaternisée ou de la cellulose quaternisée.

On a indiqué plus haut, à titre d'exemple, la composition d'un tel produit, mais il faut remarquer que, de façon tout à fait surprenante, les lotions se trouvant dans le commerce et fournies aux salons de coiffure comme produit démêlant et,

4

d'une façon générale, de conditionnement avant permanente, convenaient parfaitement comme produit de rasage.

Les avantages de l'utilisation d'une lotion liquide à la place d'une mousse ou d'une crème sont assez nombreux :

Du fait que le produit, contenant au moins 90 % d'eau, est un liquide, il suffit d'une très petite quantité pour mouiller la peau, l'effet de ramollissement des poils étant immédiat car le liquide est directement en contact avec la peau. De ce fait, une petite quantité sera suffisante pour un grand nombre d'utilisations et le produit pourra être présenté dans un flacon de dimension assez réduite qui trouvera facilement sa place, par exemple dans une trousse de toilette, ce qui n'était pas le cas des bombes de mousse, ni même des tubes de crème à raser.

Le produit liquide s'étale sur la peau sous forme d'un film très mince et transparent qui ne cache pas les poils et même, en les mouillant, renforce le contraste, ce qui permet de mieux les distinguer. On contrôlera donc très facilement l'effet du passage du rasoir et, comme le liquide est directement au contact de la peau, il n'est pas totalement enlevé par le rasoir et l'on peut donc repasser celui-ci sans gêne en cas de besoin.

A la fin du rasage, le rinçage est inutile et, grâce à son effet adoucissant sur la peau, le produit sert également de lotion après rasage en l'étalant simplement de nouveau avec la main. En cas de besoin, il suffit d'essuyer le visage avec une serviette .

0204638

5

Le produit selon l'invention sera donc particulièrement pratique pour le voyage mais, en raison de sa facilité d'emploi et de son agrément, il sera utilisé avantageusement en toutes circonstances. Il présente d'ailleurs, en outre, l'avantage d'être plus économique du fait qu'il peut être présenté dans de simples flacons, que l'on utilise beaucoup moins de produit puisqu'il se répartit très facilement et qu'il n'y a pas de pertes et que, étant du même type que certaines lotions capillaires, il peut être fabriqué en grande quantité. Toutefois, si certaines lotions capillaires existantes peuvent directement servir de lotion de rasage, il est bien certain que l'on pourrait, pour différentes raisons, en modifier la composition sans sortir du cadre de protection revendiquée, l'invention n'étant évidemment pas limitée à la seule composition qui n'a été donnée plus haut qu'à titre d'exemple.

REVENDICATIONS

1. Produit de rasage constitué par une lotion liquide destinée à être appliquée sur la peau du visage pour faciliter le passage d'un rasoir à lame, caractérisé par le fait qu'il est réalisé à partir d'un produit de conditionnement des cheveux facilitant le démêlage, contenant un produit capillaire adoucissant du cheveu et ayant de l'affinité pour la kératine, un produit adoucissant de la peau et un produit mouillant et dont la teneur en eau et la viscosité sont adaptées pour former une lotion susceptible d'être étalée à la main en formant un film sur le visage.

2. Produit de rasage selon la revendication 1, caractérisé par le fait qu'il contient :

- de 4,5 à 5 % d'une cire auto-émulsionnable,
- de 0,2 à 0,5 % d'un polymère quaternaire,
- de 0,5 à 1 % d'un produit tensio-actif,
- au moins 90 % d'eau,

et un produit épaississant en quantité réglée en fonction des proportions des autres constituants de telle sorte que la viscosité du produit de rasage soit comprise entre 50 et 500 centipoises.

3. Utilisation d'une lotion de traitement des cheveux contenant un produit démêlant, comme produit de rasage susceptible d'être appliqué sur le visage sans formation préalable de mousse pour faciliter le passage du rasoir en laissant voir le poil par transparence, et d'être éliminée sans rinçage par simple essuyage, le reste du produit servant de lotion d'après-rasage.

**0204638**

Numero de la demande

**RAPPORT DE RECHERCHE EUROPEENNE**

Office européen
des brevets

EP 86 40 1204

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| X,Y | FR-A-1 354 906 (F.J. ERTZINGER) <br> * Page 1, colonne 1, alinéas 5,6; page 1, colonne 2, alinéa 2; revendications * | 1-3 | A 61 K 7/15 |
| X,Y | DE-A-3 307 028 (H. STEINBERGER) <br> * Revendications; page 3, exemple * | 1,2 | |
| Y | US-A-4 457 912 (N.F: SCODARI) <br> * Colonne 2, ligne 38 - colonne 5, ligne 54; revendications * | 1,3 | |
| X | BE-A- 726 995 (C. DIMOU et al.) <br> * En entier * | 1,3 | |
| X | US-A-3 072 535 (A.J. MUELLER) <br> * Colonne 1, lignes 28-43; colonne 3, ligne 21 - colonne 4, ligne 42; revendications * | 1-3 | DOMAINES TECHNIQUES RECHERCHES (Int. Cl 4) <br><br> A 61 K |
| X | EP-A-0 034 126 (L. MAGGESI) <br> * Page 5, ligne 11 - page 7, ligne 14; page 9, ligne 19 - page 12, ligne 5; exemples 1,2; revendications * | 1-3 | |

Le present rapport de recherche a été établi pour toutes les revendications

| Lieu de la recherche | Date d'achèvement de la recherche | Examinateur |
|---|---|---|
| LA HAYE | 02-09-1986 | BERTE M.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié a la date de dépôt ou après cette date
D : cité dans la demande
L : cité pour d'autres raisons

& : membre de la même famille, document correspondant

OEB Form 1503 03 82